(19) Europäisches Patentamt
European Patent Office
Office européen des brevets

(11) **EP 3 276 342 B1**

(12) **EUROPÄISCHE PATENTSCHRIFT**

(45) Veröffentlichungstag und Bekanntmachung des Hinweises auf die Patenterteilung:
**08.03.2023   Patentblatt 2023/10**

(21) Anmeldenummer: **16181353.0**

(22) Anmeldetag: **27.07.2016**

(51) Internationale Patentklassifikation (IPC):
**G01N 30/86** (2006.01)        G01N 30/62 (2006.01)
**G01N 30/46** (2006.01)        G01N 30/04 (2006.01)

(52) Gemeinsame Patentklassifikation (CPC):
**G01N 30/8665; G01N 30/8658;** G01N 30/461;
G01N 2030/045; G01N 2030/625

(54) **VERFAHREN ZUM KALIBRIEREN EINES GASCHROMATOGRAPHEN**

METHOD FOR CALIBRATING A GAS CHROMATOGRAPH

PROCÉDÉ D'ÉTALONNAGE D'UN CHROMATOGRAPHE EN PHASE GAZEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.01.2018   Patentblatt 2018/05**

(73) Patentinhaber: **Siemens Aktiengesellschaft 80333 München (DE)**

(72) Erfinder: **Strauch, Piotr 76761 Rülzheim (DE)**

(74) Vertreter: **Siemens Patent Attorneys Postfach 22 16 34 80506 München (DE)**

(56) Entgegenhaltungen:
**US-A1- 2003 066 803      US-A1- 2012 016 597 US-A1- 2014 260 509**

- **KAREN ROME, ALLYSON MCINTYRE: "Intelligent use of relative response factors in gas chromatography-flame ionisation detection", CHROMATOGRAPHY TODAY, 1. Mai 2012 (2012-05-01), Seiten 52-56, XP002763884,**

**Beschreibung**

Die Erfindung betrifft ein Verfahren zum Kalibrieren eines Gaschromatographen und einen Gaschromatographen.

Wie z. B. aus der WO 03/083467 A2 bekannt, wird bei der gaschromatographischen Analyse eines Stoffgemischs eine dosierte Probe des (gasförmigen oder erforderlichenfalls verdampften) Stoffgemischs mit Hilfe eines Trägergases durch eine chromatographische Trenneinrichtung geleitet. Dabei werden die Komponenten des Stoffgemischs aufgrund unterschiedlicher Retentionszeiten getrennt, so dass sie nacheinander am Ausgang der Trenneinrichtung erscheinen. Dort werden die einzeln austretenden Komponenten mittels eines geeigneten Detektors detektiert. Dazu stehen unterschiedliche Typen von Detektoren wie z. B. Wärmeleitfähigkeitsdetektoren oder Flammenionisationsdetektoren zur Verfügung. Der in den meisten Fällen verwendete Wärmeleitfähigkeitsdetektor vergleicht die Wärmeleitfähigkeit der aktuell detektierten Komponente mit der des Trägergases und erzeugt dabei eine Detektorantwort in Form eines (im Idealfall gaussförmigen) Peaks, dessen Fläche normalerweise proportional zu der Menge der detektierten Komponente ist. Über die Dauer der Analyse des Stoffgemischs erzeugt der Detektor somit ein Chromatogramm, in dem die örtlichen oder zeitlichen Positionen der Peaks die unterschiedlichen Komponenten und die Peakflächen (oder bei vereinfachender Betrachtung die Peakhöhen) die Mengen der Komponenten in der dem Gaschromatographen aufgegebenen Probe des Stoffgemischs bezeichnen. Durch Auswertung des Chromatogramms werden die Konzentrationen der unterschiedlichen Komponenten in dem Stoffgemisch bestimmt.

Während zur Trennung einfacher Stoffgemische eine Trennsäule als Trenneinrichtung ausreicht, erfordern komplexe Stoffgemische eine Säulenschaltung, bei der zwei oder mehr Trennsäulen mit verschiedenen Trenneigenschaften hintereinander in Reihe und ggf. parallel geschaltet sind. In diesem Fall können den einzelnen Trennsäulen Detektoren (i. d. R. Wärmeleitfähigkeitsdetektoren) nachgeschaltet sein, die die eluierenden Komponenten zerstörungsfrei detektieren und zusammen eine Detektoreinrichtung bilden.

Die Detektorantwort, insbesondere die zur quantitativen Bestimmung der detektierten Komponente dienende Peakfläche, ist primär von der Komponente, ihrer Menge und dem jeweils verwendeten Detektor abhängig. Darüber hinaus wird die Detektorantwort von unterschiedlichen Messbedingungen wie Temperatur, Druck und Durchflussrate des Trägergases, Betriebsspannung des Detektors, Verstärkung des Detektorrohsignals usw. beeinflusst.

Es ist daher erforderlich, den Gaschromatographen für die unterschiedlichen zu bestimmenden Komponenten des jeweils zu analysierenden Gasgemisches zu kalibrieren.

Die Kalibrierung kann für jede zu bestimmende Komponente einzeln erfolgen, indem dem Gaschromatographen eine Kalibrierprobe aufgegeben wird, die die jeweilige Komponente in einer bekannten Konzentration $c_i$ enthält. Damit lässt sich für den Detektor ein absoluter Responsefaktor $RF_i = A_{i,cal}/c_{i,cal}$ ermitteln, wobei $c_{i,cal}$ die Konzentration der Komponente i in der Kalibrierprobe und $A_{i,cal}$ die resultierende Detektorantwort in Form der Peakfläche bezeichnet. Unter der Voraussetzung, dass bei der Analyse eines die Komponente i in unbekannter Konzentration enthaltenden Gasgemischs dieselben Messbedingungen wie bei der Kalibrierung vorliegen (wobei insbesondere auch die dosierte Probenmenge dieselbe ist) und sich der Detektor über den Konzentrationsmessbereich linear verhält, kann die Konzentration $c_i$ der Komponente i unter Verwendung des ermittelten absoluten Responsefaktors $RF_i$ aus der Detektorantwort $A_i$ mit $c_i = A_i/RF_i$ bestimmt werden. Bei nichtlinearem Verhalten des Detektors muss die Kalibrierung mit mehreren Kalibrierproben mit unterschiedlichen bekannten Konzentrationswerten der Komponente erfolgen, um für den Detektor eine Kalibrierkurve zu erstellen.

Da es sehr schwierig ist, dieselbe Probenmenge jedes Mal mit hoher Genauigkeit reproduzierbar zu dosieren, ist es bekannt, der Kalibrierprobe und den zu analysierenden Proben von Gasgemischen jeweils einen internen Standard IS in einer bestimmten Konzentration $c_{IS}$ hinzuzufügen. Nachdem bei der Kalibrierung die Responsefaktoren $RF_i$ und $RF_{IS} = A_{IS}/c_{IS}$ für die Komponente i und den Standard IS ermittelt wurden, lässt sich bei der Analyse die Konzentration $c_i$ der Komponente i aus der Detektorantwort $A_i$ mit $c_i = c_{IS} \cdot (A_i \cdot RF_{IS})/(RF_i \cdot A_{IS})$ bestimmen. Die unbekannte Konzentration $c_i$ wird also im Verhältnis zu der bekannten Konzentration $c_{IS}$ gemessen, welches Verhältnis von der jeweils dosierten Probenmenge unabhängig ist.

Der Responsefaktor $RF_i$ der Komponente i kann in Bezug zu dem Responsefaktor $RF_{IS}$ des Standards IS gesetzt werden, so dass man einen relativen Responsefaktor $RRF_{i-IS} = RF_i/RF_{IS}$ erhält, anhand dessen sich die Konzentration $c_i$ der Komponente i mit $c_i = c_{IS} \cdot (1/RRF_{i-IS}) \cdot (A_i/A_{IS})$ bestimmen lässt.

Relative Responsefaktoren können allgemein dazu verwendet werden, eine unbekannte Konzentration $c_i$ einer ersten Komponente i eines Stoffgemischs in Gegenwart einer zweiten Komponente (Bezugskomponente) k zu bestimmen, deren Konzentration $c_k$ bekannt ist. Damit ist es möglich, auch die Konzentrationen $c_i$ von Komponenten i zu messen, für die kein eigener Responsefaktor $RF_i$ vorliegt bzw. durch Kalibration ermittelt wurde: $c_i = c_k \cdot (1/RRF_{i-k}) \cdot (A_i/A_k) = A_i/(RF_k \cdot RRF_{i-k})$.

Auch die relativen Responsefaktoren $RRF_{i-k}$ müssen im Rahmen einer Kalibration ermittelt werden. Der Vorteil ihrer Verwendung liegt darin, dass sie in einem sinnvollen Geltungsbereich von der dosierten Probenmenge (Dosiermenge) und den Messbedingungen unabhängig sind; dem Kalibriergemisch können neue Komponenten hinzugefügt werden, und es ist möglich die relativen Responsefaktoren auf andere Bezugskomponenten umzurechnen; also z. B.: $RRF_{j-k} = RRF_{j-i} \cdot RRF_{i-k}$. Die Konzentration $c_j$ einer Komponente j lässt sich also bei nicht bekanntem relativem Responsefaktor $RRF_{j-k}$ aber bekannten relativen Responsefaktoren $RRF_{j-i}$ und $RRF_{i-k}$ wie folgt bestimmen:

$$ c_j \;=\; c_k \cdot \left(1/\left(RRF_{j-i} \cdot RRF_{i-k}\right)\right) \cdot \left(A_j/A_k\right) \;=\; A_j/\left(RF_k \cdot RRF_{j-i} \cdot RRF_{i-k}\right). $$

Responsefaktoren können auch anderweitig definiert und berechnet werden, beispielsweise als Kehrwert der oben erläuterten Faktoren. Zum Stand der Technik wird auf folgende Literaturstellen verwiesen:
"Guidelines for the quantitative gas chromatography of volatile flavouring substances, from the Working Group on Methods of Analysis of the International Organization of the Flavor Industry (IOFI)", Flavour and Fragrance Journal, 2011, 26, 297-299.

K. Rome et al.: "Intelligent use of Relative Response Factors in Gas Chromatography-Flame Ionisation Detection", Chromatography Today, May/June 2012, 52-56.

Kalibrationsverfahren für Chromatographen sind auch aus der US 2014/260509 A1 oder US 2003/066803 A1 bekannt.

Die US 2014/260509 A1 offenbart einen Flüssigchromatographen, in den zum Kalibrieren eine Standard-Lösung mit mindestens zwei Analyten in unterschiedlichen bekannten Konzentrationen injiziert wird, so dass als Detektorantwort Peaks mit unterschiedlichen Peakhöhen oder -flächen und unterschiedlichen Retentionszeiten erhalten werden. Die beiden Analyten werden unabhängig von der Reihenfolge, in der sie aus chromatographischen Trenneinrichtung austreten und damit unabhängig von der verwendeten Trenneinrichtung oder ihrem Zustand anhand des Verhältnisses ihrer Peakhöhen bzw. -flächen identifiziert. Bei einer Re-Kalibration werden die für die beiden Analyten erhaltenen Retentionszeiten daraufhin überwacht, ob sie sich gegenüber der Erst-Kalibration um mehr als ein vorgegebenes Maß geändert haben; ist dies der Fall, wird eine Meldung erzeugt, das die Trenneinrichtung ausgetauscht werden muss.

Die US 2003/066803 A1 beschreibt ein Verfahren zum Kalibrieren eines Lichtstreudetektors (ELSD - Evaporative Light Scattering Detector), der in Verbindung mit einem Flüssigchromatographen genutzt wird. Ein Lösungsmittel mit darin gelösten und chromatographisch getrennten Komponenten eines Stoffgemischs wird in dem Detektor vernebelt und verdampft. Die dabei entstehenden Feststoffpartikel der Komponenten werden durch einen Lichtstrahl geleitet, und das an den Partikeln gestreute Licht wird detektiert. Die Detektorantwort ist ein Peak mit einer zu der Masse der detektierten Komponente proportionalen Fläche und darüber hinaus von der Zusammensetzung des Lösungsmittels abhängig. Zur Kalibration des Detektors werden in diesen Proben eines Lösungsmittel mit darin gelösten Feststoffpartikeln injiziert, wobei die organische Zusammensetzung des Lösungsmittels, die Feststoffsubstanzen und die Feststoffmasse variiert werden. Die Kalibrationsdaten einschließlich der ermittelten Peakflächen werden abgespeichert. Bei der chromatographischen Analyse eines Stoffgemischs werden die erhaltenen Peakflächen und die bekannte Zusammensetzung des verwendeten Lösungsmittels mit den Kalibrationsdaten verglichen, um unmittelbar aus den Kalibrationsdaten oder durch Interpolation zwischen ihnen die Massen der gesuchten Komponenten des Stoffgemischs zu ermitteln.

Fehler bei der Kalibrierung eines Gaschromatographen im Allgemeinen oder Gaschromatographen im Speziellen können die Genauigkeit der chromatographischen Analyse erheblich beeinträchtigen. So können aufgrund von Fehlern seitens des Anwenders, beispielsweise aufgrund einer Verwechslung, falsche Kalibriergemische verwendet werden oder, z. B. bei Eingabefehlern, falsche Informationen über das Kalibriergemisch in die Auswerteeinrichtung des Gaschromatographen übertragen werden. Das Kalibriergemisch selbst kann sich aufgrund von Undichtigkeit der Gasflasche, Verunreinigung, Alterung verändert haben, so dass es nicht mehr der Spezifikation entspricht. Auch kann die Parametrierung des Gaschromatographen verändert worden sein. Weitere Fehlerquellen sind z. B. Undichtigkeiten und fehlerhafte Spülung der Gaswege des Gaschromatographen.

Der Erfindung liegt daher die Aufgabe zugrunde, die Kalibrierung eines Gaschromatographen fehlersicherer zu machen.

Gemäß der Erfindung wird die Aufgabe durch das in Anspruch 1 definierte Verfahren und den Gaschromatographen nach Anspruch 7 gelöst.

Vorteilhafte Weiterbildungen des erfindungsgemäßen Verfahrens bzw. Gaschromatographen sind in den Unteransprüchen angegeben.

Gegenstand der Erfindung ist somit ein Verfahren zum Kalibrieren eines Gaschromatographen, der eine Dosiereinrichtung, eine Trenneinrichtung, eine Detektoreinrichtung und eine Auswerteeinrichtung umfasst, die dazu angeordnet und ausgebildet sind, eine Probe eines zu analysierenden Stoffgemischs zu dosieren, die dosierte Probe zur Trennung von in dem Stoffgemisch enthaltenen Komponenten durch die Trenneinrichtung zu leiten, an deren Ende ausgewählte getrennte Komponenten zu detektieren und ihre Konzentrationen in dem Stoffgemisch anhand von der Detektoreinrichtung gelieferter Detektorantworten und in der Auswerteeinrichtung gespeicherten Responsefaktoren quantitativ zu bestimmen, wobei die Bestimmung der Konzentration mindestens einer ersten Komponente des Stoffgemischs in Abhängigkeit von der Detektorantwort auf diese Komponente, der bestimmten oder bekannten Konzentration einer zweiten Komponente des Stoffgemischs und eines relativen Responsefaktors erfolgt,

- wobei bei der Kalibrierung eine oder mehrere Proben eines oder mehrerer Kalibriergemische, die die Komponenten in bekannten Konzentrationen enthalten, in dem Gaschromatographen analysiert wird und die relativen Responsefaktoren anhand der erhaltenen Detektorantworten und der bekannten Konzentrationen ermittelt und in der Auswerteeinrichtung abgespeichert werden,

dadurch gekennzeichnet,

- dass bei der Kalibrierung die ermittelten relativen Responsefaktoren in der Auswerteeinrichtung mit für die Detektoreinrichtung typischen universellen relativen Responsefaktoren verglichen werden, und
- dass durch die Auswerteeinrichtung eine Fehlermeldung erzeugt und ausgegeben wird, wenn die bei der Kalibration ermittelten relativen Responsefaktoren von den universellen relativen Responsefaktoren über ein vorgegebenes Maß hinaus abweichen, wobei die universellen relativen Responsefaktoren unveränderlich sind.

Der Ausdruck "Responsefaktor" bzw. "relativer Responsefaktor" ist nicht nur im engen Sinne eines einzelnen Faktors zu verstehen, sondern kann, insbesondere bei nichtlinearem Verhalten des Detektors, auch eine "Responsefunktion" bzw. "relative Responsefunktion" über den Konzentrationsmessbereich darstellen.

Der Erfindung liegt die Erkenntnis zugrunde, dass die in der Gaschromatographie heutzutage zum Einsatz kommenden Detektoren aufgrund von Serienfertigung eine sehr hohe Reproduzierbarkeit aufweisen. Dies gilt insbesondere für mikrotechnisch hergestellte (MEMS-)Detektoren, wie z. B. Wärmeleitfähigkeitsdetektoren. Dank dieser hohen Reproduzierbarkeit sind die relativen Responsefaktoren der Detektoren unveränderlich und haben einen universellen Charakter. Die universellen relativen Responsefaktoren können daher, z. B. beim Hersteller der Gaschromatographen und/oder Detektoren, für unterschiedliche Komponenten ermittelt und den Detektoren, beispielsweise in Form einer Gerätebeschreibung, mitgegeben werden, oder elektronisch abrufbar, beispielsweise auf einem im Internet erreichbaren entfernten Rechner (Cloud), bereitgestellt werden. Die Gerätebeschreibung mit den universellen relativen Responsefaktoren kann auf einem Datenträger hinterlegt sein, der von der Auswerteeinrichtung des Gaschromatographen drahtgebunden und/oder drahtlos ausgelesen werden kann. Beispielsweise kann der Datenträger als Speicherchip, z. B. in Form eines RFID-Tags, an der Detektoreinrichtung bzw. den diese bildenden Detektoren ausgebildet sein.

Für den Fall, dass über das vorgegebene Maß hinausgehende Abweichungen bei mehreren Komponenten festgestellt werden, kann die Auswerteeinrichtung ein Abweichungsmuster erkennen und anhand dessen eine Fehlerursache melden. Dies kann durch Vergleich mit vorgegebenen Abweichungsmustern erfolgen, denen unterschiedliche Fehler zugeordnet sind.

Die Erfindung ermöglicht es dem Anwender mögliche Fehler bei der Kalibrierung zu signalisieren und ihm ggf. auch Hinweise auf Fehlerursachen zu geben.

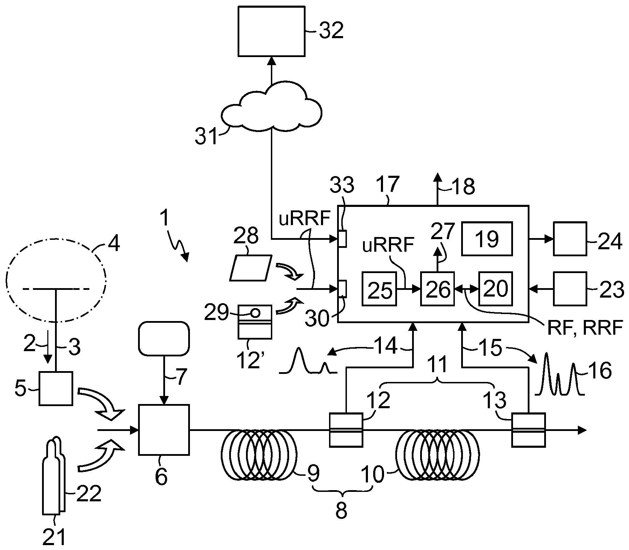

Zur weiteren Erläuterung der Erfindung wird im Folgenden auf die einzige Figur der Zeichnung Bezug genommen, die ein Beispiel für einen Gaschromatographen zeigt.

Der sehr vereinfacht dargestellte Gaschromatograph 1 dient zur Analyse eines Stoffgemischs 2, das über eine Leitung 3 aus einem technischen Prozess 4 entnommen wird. Das Stoffgemisch 2 wird ggf. in einer Aufbereitungseinrichtung 5 für die chromatographische Analyse aufbereitet, beispielsweise verdampft, bevor es dem Gaschromatographen 1 aufgegeben wird. In einer Dosiereinrichtung 6 wird aus dem Stoffgemisch 2 eine Probe in Form eines möglichst kurzen und scharf begrenzten Dosierpfropfs ausgeschleust und anschließend mittels eines Trägergases 7 durch eine Trenneinrichtung 8 geleitet. Die Trenneinrichtung 8 kann in bekannter Weise aus einer einzelnen Trennsäule oder, wie hier gezeigt, aus einer Schaltung von zwei oder mehr Trennsäulen 9, 10 bestehen. Beim Durchströmen der Trenneinrichtung 8 werden die in der dosierten Probe enthaltenen Komponenten des Stoffgemischs 2 getrennt, so dass sie zeitlich nacheinander aus der Trenneinrichtung 8 austreten und mittels einer Detektoreinrichtung 11 detektiert werden. Bei dem gezeigten Beispiel weist die Detektoreinrichtung 11 zwei Detektoren 12, 13 auf, wobei der Detektor 12 eine vorgegebene Anzahl der aus der Trennsäule 9 austretenden und bis dahin ausreichend getrennten Komponenten detektiert und der Detektor 13 die übrigen Komponenten nach ihrer Trennung in der Trennsäule 10 detektiert. Ggf. können die von dem Detektor 12 detektierten Komponenten vor Erreichen der Trennsäule 10 aus der Trenneinrichtung 8 ausgeschleust werden.

Die Detektoren 12, 13 liefern als Detektorsignal 14 bzw. 15 jeweils ein Chromatogramm, in dem für jede detektierte Komponente eine Detektorantwort in Form eines Peaks, z. B. 16, erscheint, dessen Höhe und Flächeninhalt von einer detektionsspezifischen Eigenschaft der Komponente, ihrer Menge in der Probe und dem verwendeten Detektor abhängig ist. In dem gezeigten Ausführungsbeispiel handelt es sich bei den Detektoren 12, 13 um mikrotechnisch hergestellte (MEMS-)Wärmeleitfähigkeitsdetektoren, so dass die detektionsspezifische Eigenschaft der Komponente deren Wärmeleitfähigkeit im Vergleich zu der des Trägergases 7 ist. Die Chromatogramme 14, 15 werden in einer der Detektoreinrichtung 8 nachgeordneten Auswerteeinrichtung 17 ausgewertet, um ausgewählte Komponenten des zu analysierenden Stoffgemischs 2 quantitativ zu bestimmen und schließlich als Analysenergebnis 18 auszugeben. Dabei wird die Konzentration einer jeden Komponenten in einer Recheneinheit 19 in Abhängigkeit von der jeweiligen Detektorantwort (Peak) 16 auf diese Komponente und einem Responsefaktor berechnet, der im Rahmen einer Kalibration für die betreffende Komponente ermittelt wurde und in einem Kalibrationsdatenspeicher 20 abgelegt wurde.

Wie eingangs bereits erläutert, beschreiben die Responsefaktoren allgemein die Reaktion des Detektors auf unterschiedliche Komponenten, also in einem einzelnen Fall den Zusammenhang zwischen der Konzentration einer bestimmten detektierten Komponente und der daraus resultierenden Detektorantwort. Dieser Zusammenhang kann ein einzelner Wert oder eine von der Konzentration abhängige Funktion sein. Typischerweise geben absolute Responsefaktoren RF das Verhältnis von Detektorantwort und Konzentration an. Relative Responsefaktoren RRF werden verwendet, um die Reaktion des Detektors auf eine bestimmte Komponente in Bezug auf die Reaktion des Detektors auf eine andere bestimmte Komponente zu beschreiben. Typischerweise gibt ein relativer Responsefaktor RRF das Verhältnis zweier Responsefaktoren RF für unterschiedliche Komponenten an. Verhält sich der Detektor gegenüber beiden Komponenten gleich, so ist der relative Responsefaktor RRF gleich eins. Relative Responsefaktoren RRF können aus absoluten und/oder relativen Responsefaktoren RF, RRF gebildet werden.

Die Responsefaktoren RF, RRF werden im Rahmen einer Kalibration ermittelt, wobei dem Gaschromatographen 1 Proben von Kalibriergemischen 21, 22 aufgegeben werden, die interessierende Komponenten oder Gemische der Komponenten mit vorgegebenen Konzentrationen enthalten. Aus den bei der Analyse der Kalibrierproben für die unterschiedlichen Komponenten erzeugten Detektorantworten (Peaks) 16 und den zugehörigen bekannten Konzentrationswerten, die von dem Anwender in die Auswerteeinrichtung 17 eingegeben werden, berechnet die Auswerteeinrichtung 17 die Responsefaktoren RF, RRF und legt diese in dem Kalibrationsdatenspeicher 20 ab. Dazu stehen dem Anwender Mittel zur Eingabe 23 der bekannten Konzentrationswerte und Visualisierung 24 der von der Auswerteeinrichtung 17 aufbereiteten Detektorantworten (z. B. Tastatur und Display, Touchscreen, externer PC) zur Verfügung. Die Ermittlung und Abspeicherung der Responsefaktoren RF, RRF wird nach Bedarf von dem Anwender unterstützt und überwacht bzw. erfolgt automatisch. Insoweit dies z. B. über einen externen PC oder dergleichen erfolgt, kann dieser im Sinne der Erfindung als Bestandteil der Auswerteeinrichtung 17 angesehen werden. Die Auswerteeinrichtung (Elektronikteil) 17 kann auch räumlich getrennt von dem Analysenteil des Gaschromatographen 1 angeordnet sein.

Die Auswerteeinrichtung 17 weist einen weiteren Speicher 25 auf, in dem universelle relative Responsefaktoren uRRF gespeichert sind, die für die verwendeten Detektoren oder Detektortypen 12, 13 sowie unterschiedliche Komponenten einmalig ermittelt worden sind. Die während der Kalibrierung ermittelten relativen Responsefaktoren RRF werden in einer Vergleichseinrichtung 26 der Auswerteeinrichtung 17 mit den universellen relativen Responsefaktoren uRRF verglichen, wobei eine Fehlermeldung 27 erzeugt wird, sobald bei der Kalibration ermittelte relative Responsefaktoren RRF von den zugehörigen universellen relativen Responsefaktoren uRRF um ein vorgegebenes Maß abweichen. Da, wie weiter oben bereits erläutert, relative Responsefaktoren für bestimmte Komponenten auf andere Bezugskomponenten umgerechnet werden können, umfasst der Vergleich auch solche nicht unmittelbar durch Messung sondern rechnerisch ermittelte relative Responsefaktoren RRF und/oder universelle relative Responsefaktoren uRRF. Die Fehlermeldung 27 und vorzugsweise auch der Betrag der Abweichung werden dem Anwender mitgeteilt, z. B. auf den Visualisierungsmitteln 24 angezeigt. Auf diese Weise werden dem Anwender mögliche Fehler bei der Kalibrierung signalisiert und ggf. auch Hinweise auf Fehlerursachen gegeben. Wenn z. B. bei allen Komponenten eine Kalibriergemischs, z. B. 22, eine überhöhte Abweichung zwischen den ermittelten relativen Responsefaktoren RRF und den zugehörigen universellen relativen Responsefaktoren uRRF auftritt, so kann dies auf einen Fehler bei dem Kalibriergemisch 22 hinweisen. Kommt es bei mehreren oder allen Kalibriergemischen 21, 22 zu Abweichungen, kann ein Fehler bei dem Gaschromatographen 1 oder seiner Bedienung vorliegen. Singuläre Abweichungen bei einzelnen Komponenten weisen auf einen möglichen Eingabefehler seitens des Anwenders hin. Anhand weiterer Abweichungsmuster kann die Auswerteeinrichtung 17 z. B. eine Verwechslung der verwendeten Kalibriergemische als möglichen Fehler identifizieren und dem Anwender mitteilen.

Die in dem Gaschromatographen 1 verwendeten Detektoren 12, 13, insbesondere die hier beispielhaft verwendeten mikrotechnisch hergestellten Wärmeleitfähigkeitsdetektoren, weisen aufgrund von Serienfertigung eine sehr hohe Reproduzierbarkeit auf. Dank dieser hohen Reproduzierbarkeit sind die relativen Responsefaktoren RRF der Detektoren 12, 13 unveränderlich und haben einen universellen Charakter. Die universellen relativen Responsefaktoren uRRF können daher z. B. herstellerseitig für unterschiedliche Komponenten ermittelt und den Detektoren, beispielsweise in Form einer Gerätebeschreibung mitgegeben werden. Bei dem gezeigten Ausführungsbeispiel geschieht dies z. B. über einen separaten Datenträger 28, z. B. USB-Stick, oder einen Datenträger, z. B. Speicherchip 29, auf dem Detektor 12', in welche die universellen relativen Responsefaktoren uRRF eingeschrieben werden. Diese Informationen können über eine geeignete Datenschnittstelle 30 der Auswerteeinrichtung 17 drahtgebunden oder drahtlos ausgelesen und in den Speicher 25 übertragen werden. Ergänzend oder alternativ können die die universellen relativen Responsefaktoren uRRF auf einem im Internet 31 erreichbaren entfernten Rechner 32 (Cloud), bereitgestellt werden.

**Patentansprüche**

1. Verfahren zum Kalibrieren eines Gaschromatographen (1), der eine Dosiereinrichtung (6), eine Trenneinrichtung (8), eine Detektoreinrichtung (11) und eine Auswerteeinrichtung (17) umfasst, die dazu angeordnet und ausgebildet sind, eine Probe eines zu analysierenden Stoffgemischs (2) zu dosieren, die dosierte Probe zur Trennung von in dem Stoffgemisch (2) enthaltenen Komponenten durch die Trenneinrichtung (8) zu leiten, an deren Ende ausgewählte getrennte Komponenten zu detektieren und ihre Konzentrationen in dem Stoffgemisch (2) anhand von der Detektoreinrichtung (11) gelieferter Detektorantworten (6) und in der Auswerteeinrichtung (17) gespeicherten Responsefaktoren (RF, RRF) quantitativ zu bestimmen, wobei die Bestimmung der Konzentration mindestens einer ersten Komponente des Stoffgemischs (2) in Abhängigkeit von der Detektorantwort (16) auf diese Komponente, der bestimmten oder bekannten Konzentration einer zweiten Komponente des Stoffgemischs (2) und eines relativen Responsefaktors (RRF) erfolgt,

   - wobei bei der Kalibrierung eine oder mehrere Proben eines oder mehrerer Kalibriergemische (21, 22), die die Komponenten in bekannten Konzentrationen enthalten, in dem Gaschromatographen (1) analysiert wird und die relativen Responsefaktoren (RRF) anhand der erhaltenen Detektorantworten (16) und der bekannten Konzentrationen ermittelt und in der Auswerteeinrichtung (17) abgespeichert werden,

   **dadurch gekennzeichnet,**

   - **dass** bei der Kalibrierung die ermittelten relativen Responsefaktoren (RRF) in der Auswerteeinrichtung (17) mit für die Detektoreinrichtung (11) typischen universellen relativen Responsefaktoren (uRRF) verglichen werden, und
   - **dass** durch die Auswerteeinrichtung (17) eine Fehlermeldung (27) erzeugt und ausgegeben wird, wenn die bei der Kalibration ermittelten relativen Responsefaktoren (RRF) von den universellen relativen Responsefaktoren (uRRF) über ein vorgegebenes Maß hinaus abweichen,

   wobei die universellen relativen Responsefaktoren(uRRF) unveränderlich sind.

2. Verfahren nach Anspruch 1, **dadurch gekennzeichnet, dass** die universellen relativen Responsefaktoren (uRRF) von dem Hersteller der Detektoreinrichtung (11) und/oder des Gaschromatographen (1) ermittelt und bereitgestellt werden.

3. Verfahren nach Anspruch 1 oder 2, dass die universellen relativen Responsefaktoren (uRRF) in elektronischer Form bereitgestellt werden.

4. Verfahren nach Anspruch 3, **dadurch gekennzeichnet, dass** die universellen relativen Responsefaktoren (uRRF) auf einem im Internet (31) erreichbaren entfernten Rechner (32) bereitgestellt und von diesem abgerufen werden.

5. Verfahren nach Anspruch 1, 2 oder 3, **dadurch gekennzeichnet, dass** die universellen relativen Responsefaktoren (uRRF) der Detektoreinrichtung (11) als Gerätebeschreibung mitgegeben werden.

6. Verfahren nach einem der vorangehenden Ansprüche, **dadurch gekennzeichnet, dass** die Auswerteeinrichtung (17) für den Fall, dass über das vorgegebene Maß hinausgehenden Abweichungen bei mehreren Komponenten festgestellt werden, ein Abweichungsmuster erkennt und anhand dessen eine Fehlerursache meldet.

7. Gaschromatograph (1), der eine Dosiereinrichtung (6), eine Trenneinrichtung (8), eine Detektoreinrichtung (11) und eine Auswerteeinrichtung (17) mit einem Kalibrationsdatenspeicher (20) umfasst, die dazu angeordnet und ausgebildet sind, eine Probe eines zu analysierenden Stoffgemischs (2) zu dosieren, die dosierte Probe zur Trennung von in dem Stoffgemisch (2) enthaltenen Komponenten durch die Trenneinrichtung (8) zu leiten, an deren Ende ausgewählte getrennte Komponenten zu detektieren und ihre Konzentrationen in dem Stoffgemisch (2) anhand von der Detektoreinrichtung (11) gelieferter Detektorantworten (6) und in der Auswerteeinrichtung (17) gespeicherten Responsefaktoren (RF, RRF) quantitativ zu bestimmen, wobei die Bestimmung der Konzentration mindestens einer ersten Komponente des Stoffgemischs (2) in Abhängigkeit von der Detektorantwort auf diese Komponente, der bestimmten oder bekannten Konzentration einer zweiten Komponente des Stoffgemischs (2) und eines relativen Responsefaktors (RRF) erfolgt, wobei die Auswerteeinrichtung (17) ferner dazu ausgebildet ist, die relativen Responsefaktoren (RRF) bei der Kalibrierung des Gaschromatographen (1) mit einer oder mehreren Proben eines oder mehrerer die Komponenten in bekannten Konzentrationen enthaltenden Kalibriergemische (21, 22) anhand der erhaltenen Detektorantworten (16) und der bekannten Konzentrationen zu ermitteln und in dem Kalibrationsdatenspeicher (20) abzuspeichern,

**dadurch gekennzeichnet,**

**dass** die Auswerteeinrichtung (17) ferner einen Speicher (25) und eine Vergleichseinrichtung (26) enthält, die dazu ausgebildet ist, die bei der Kalibrierung ermittelten relativen Responsefaktoren (RRF) mit in dem Speicher (25) enthaltenen für die Detektoreinrichtung (11) typischen universellen relativen Responsefaktoren (uRRF) zu vergleichen und eine Fehlermeldung (27) zu erzeugen und auszugeben, wenn die bei der Kalibration ermittelten relativen Responsefaktoren (RRF) von den universellen relativen Responsefaktoren (uRRF) über ein vorgegebenes Maß hinaus abweichen, wobei die universellen relativen Responsefaktoren(uRRF) unveränderlich sind.

8. Gaschromatograph (1) nach Anspruch 7, **dadurch gekennzeichnet, dass** die Auswerteeinrichtung (17) mindestens eine Datenschnittstelle (30, 33) zum Empfang der universellen relativen Responsefaktoren (uRRF) aufweist.

9. Gaschromatograph (1) nach Anspruch 8, **dadurch gekennzeichnet, dass** die Datenschnittstelle (33) dazu ausgebildet ist, die universellen relativen Responsefaktoren (uRRF) von einem im Internet (31) erreichbaren entfernten Rechner (32) zu empfangen.

10. Gaschromatograph (1) nach Anspruch 8 oder 9, **dadurch gekennzeichnet, dass** die Datenschnittstelle (30) dazu ausgebildet ist, die universellen relativen Responsefaktoren (uRRF) drahtgebunden oder drahtlos von einem Datenträger (28, 29) auszulesen.

11. Gaschromatograph (1) nach Anspruch 10, **dadurch gekennzeichnet, dass** der Datenträger aus einem an der Detektoreinrichtung (11) angeordneten Datenträger (29), insbesondere Speicherchip, besteht.

12. Gaschromatograph (1) nach einem der vorangehenden Ansprüche, **dadurch gekennzeichnet, dass** die Auswerteeinrichtung (17) dazu ausgebildet ist, für den Fall, dass über das vorgegebene Maß hinausgehenden Abweichungen bei mehreren Komponenten festgestellt werden, ein Abweichungsmuster zu erkennen und anhand dessen eine Fehlerursache zu melden.

## Claims

1. Method for calibrating a gas chromatograph (1), which comprises a dosing device (6), a separating device (8), a detector device (11) and an evaluation device (17), which are arranged and embodied to dose a sample of a mixture of materials (2) to be analysed, to route the dosed sample in order to separate components contained in the mixture of materials (2) by means of the separating device (8), to detect selected separated components at the end thereof and to quantitatively determine their concentrations in the mixture of materials (2) on the basis of detector responses (6) supplied by the detector device (11) and response factors (RF, RRF) stored in the evaluation device (17), wherein the determination of the concentration of at least one first component of the mixture of materials (2) is carried out as a function of the detector response (16) to this component, the determined or known concentration of a second component of the mixture of materials (2) and a relative response factor (RRF),

- wherein with the calibration one or more samples of one or more calibration mixtures (21, 22), which contain the components in known concentrations, is analysed in the gaschromatograph (1) and the relative response factors (RRF) are determined on the basis of the obtained detector responses (16) and the known concentrations and are stored in the evaluation device (17),

**characterised in that**

- with the calibration, the determined relative response factors (RRF) in the evaluation device (17) are compared with universal relative response factors (uRRF) typical of the detector device (11), and
- that an error message (27) is generated and output by the evaluation device (17), if the relative response factors (RRF) determined during the calibration deviate beyond a predetermined degree from the universal relative response factors (uRRF),

wherein the universal relative response factors (uRRF) are unchangeable.

2. Method according to claim 1, **characterised in that** the universal relative response factors (uRRF) are determined and provided by the manufacturer of the detector device (11) and/or of the gas chromatograph (1).

3.  Method according to claim 1 or 2, that the universal relative response factors (uRRF) are provided in electronic form.

4.  Method according to claim 3, **characterised in that** the universal relative response factors (uRRF) are provided on a remote computer (32) which can be accessed via the internet (31) and can be retrieved herefrom.

5.  Method according to claim 1, 2, or 3, **characterised in that** the universal relative response factors (uRRF) of the detector device (11) are given as a device description.

6.  Method according to one of the preceding claims, **characterised in that** in the event that deviations extending beyond the predetermined degree are established with a number of components, the evaluation device (17) identifies a deviation pattern and on the basis of this reports an error cause.

7.  Gas chromatograph (1), which comprises a dosing device (6), a separating device (8), a detector device (11) and an evaluation device (17) with a calibration data memory (20), which are arranged and embodied to dose a sample of a mixture of materials (2) to be analysed, to route the dosed sample in order to separate components contained in the mixture of materials (2) by means of the separating device (8), to detect selected separated components at the end thereof and to quantitatively determine their concentrations in the mixture of materials (2) on the basis of detector responses (6) supplied by the detector device (11) and response factors (RF, RRF) stored in the evaluation device (17), wherein the determination of the concentration of at least one first component of the mixture of materials (2) is carried out as a function of the detector response to this component, the determined or known concentration of a second component of the mixture of materials (2) and a relative response factor (RRF), wherein the evaluation device (17) is also embodied to determine the relative response factors (RRF) while calibrating the gas chromatograph (1) with one or more samples of one or more calibration mixtures (21, 22) contained in the known concentrations on the basis of the detector responses (16) obtained and the known concentrations and to store the same in the calibration data memory (20),
    **characterised in that**
    the evaluation device (17) also contains a memory (25) and a comparison device (26), which is embodied to compare the relative response factors (RRF) determined during the calibration with universal relative response factors (uRRF) typical of the detector device (11) and contained in the memory (25) and to generate an error message (27) and to output the same if the relative response factors (RRF) determined during the calibration deviate beyond a predetermined degree from the universal relative response factors (uRRF), wherein the universal relative response factors (uRRF) are unchangeable.

8.  Gas chromatograph (1) according to claim 7, **characterised in that** the evaluation device (17) has at least one data interface (30, 33) for receiving the universal relative response factors (uRRF).

9.  Gas chromatograph (1) according to claim 8, **characterised in that** the data interface (33) is embodied to receive the universal relative response factors (uRRF) from a remote computer (32) which can be accessed via the internet (31).

10. Gas chromatograph (1) according to claim 8 or 9, **characterised in that** the data interface (30) is embodied to read out the universal relative response factors (uRRF) from a data carrier (28, 29) in a wired or wireless manner.

11. Gas chromatograph (1) according to claim 10, **characterised in that** the data carrier consists of a data carrier (29), in particular storage chip, arranged on the detector device (11).

12. Gas chromatograph (1) according to one of the preceding claims, **characterised in that** the evaluation device (17) is embodied to identify a deviation pattern and notify an error cause on this basis in the event that deviations extending beyond the predetermined degree are established with a number of components.

## Revendications

1.  Procédé d'étalonnage d'un chromatographe (1) en phase gazeuse, qui comprend un dispositif (6) de dosage, un dispositif (8) de séparation, un dispositif (11) de détection et un dispositif (17) d'évaluation, qui sont disposés et constitués pour doser un échantillon d'un mélange (2) de matière à analyser, faire passer l'échantillon dosé, pour la séparation de constituants contenus dans le mélange (2) de matière, dans le dispositif (8) de séparation, pour détecter à son extrémité des constituants séparés sélectionnés et leurs concentrations dans le mélange (2) de matière, à l'aide de réponses (6) du détecteur fournies par le dispositif (11) de détection et pour déterminer quantitativement des facteurs (RF, RRF) de réponse mis en mémoire dans le dispositif (17) d'évaluation, dans lequel la détermination de la concentration d'au moins un premier constituant du mélange (2) de matière s'effectue en fonction de la réponse (16) du détecteur à ce constituant, de la concentration déterminée ou connue d'un deuxième constituant du mélange (2) de matière et d'un facteur (RRF) de réponse relatif,

- dans lequel, lors de l'étalonnage, on analyse, dans le chromatographe (1) en phase gazeuse, un ou plusieurs échantillons d'un ou plusieurs mélanges (21, 22) d'étalonnage, qui contiennent les constituants en des concentrations connues, et on détermine les facteurs (RRF) de réponse relatifs à l'aide des réponses (16) obtenues du détecteur et des concentrations connues et on les met en mémoire dans le dispositif (17) d'évaluation,

**caractérisé**

- **en ce que**, lors de l'étalonnage, on compare les facteurs (RRF) de réponse relatifs déterminés dans le dispositif (17) d'évaluation à des facteurs (uRRF) de réponse relatifs universels typiques pour le dispositif (11) de détection, et
- **en ce que** l'on produit et émet, par le dispositif (17) d'évaluation, un message (27) d'erreur, si les facteurs (RRF) de réponse relatifs déterminés lors de l'étalonnage s'écartent de plus d'une mesure donnée à l'avance des facteurs (uRRF) de réponse relatifs universels,

dans lequel les facteurs (uRRF) de réponse relatifs universels sont invariables.

2. Procédé suivant la revendication 1, **caractérisé en ce que** l'on détermine et se procure les facteurs (uRRF) de réponse relatifs universels chez le fabricant du dispositif (11) de détection ou du chromatographe (1) en phase gazeuse.

3. Procédé suivant la revendication 1 ou 2, **caractérisé en ce que** l'on se procure les facteurs (uRRF) de réponse relatifs universels sous forme électronique.

4. Procédé suivant la revendication 3, **caractérisé en ce que** l'on se procure les facteurs (uRRF) de réponse relatifs universels sur un ordinateur (32) à distance pouvant être atteint dans l'Internet (31) et **en ce qu'**on les appelle de celui-ci.

5. Procédé suivant la revendication 1, 2 ou 3, **caractérisé en ce que** l'on donne, comme description d'appareil, les facteurs (uRRF) de réponse relatifs universels du dispositif (11) de détection.

6. Procédé suivant l'une des revendications précédentes, **caractérisé en ce que** le dispositif (17) d'évaluation reconnaît un modèle d'écart et indique une cause d'erreur à l'aide de celui-ci dans le cas où des écarts allant au-delà de la mesure donnée à l'avance sont constatés pour plusieurs constituants.

7. Chromatographe (1) en phase gazeuse, qui comprend un dispositif (6) de dosage , un dispositif (8) de séparation, un dispositif (11) de détection et un dispositif (17) d'évaluation ayant une mémoire (20) de données d'étalonnage, qui sont disposés et constitués pour doser un échantillon d'un mélange (2) de matière à analyser, faire passer l'échantillon dosé, pour la séparation de constituants contenus dans le mélange (2) de matière, dans le dispositif (8) de séparation, pour détecter à son extrémité des constituants séparés sélectionnés et pour déterminer quantitativement leurs concentrations dans le mélange (2) de matière, à l'aide de réponses (6) de détecteur fournies par le dispositif (11) de détection et de facteurs (RF, RFF) de réponse mis en mémoire dans le dispositif (17) d'évaluation, dans lequel la détermination de la concentration d'au moins un premier constituant dans le mélange (2) de matière s'effectue en fonction de la réponse de détecteur à ce constituant, de la concentration déterminée ou connue d'un deuxième constituant du mélange (2) de matière et d'un facteur (RRF) de réponse relatif,

dans lequel le dispositif (17) d'évaluation est constitué en outre pour déterminer les facteurs (RRF) de réponse relatifs, lors de l'étalonnage du chromatographe (1) en phase gazeuse avec un ou plusieurs échantillons d'un ou de plusieurs mélanges (21, 22) d'étalonnage contenant les constituants des concentrations connues, à l'aide des réponses (16) de détecteur obtenues et des concentrations connues, et pour les mettre dans la mémoire (20) de données d'étalonnage,

**caractérisé**

**en ce que** le dispositif (17) d'évaluation comporte en outre une mémoire (25) et un dispositif (26) de comparaison, qui est constitué pour comparer les facteurs (RRF) de réponse relatifs déterminés lors de l'étalonnage à des facteurs (uRRF) de réponse relatifs universels typiques du dispositif (11) de détection contenus dans la mémoire (25) et pour produire et émettre un message (27) d'erreur, si les facteurs (RRF) relatifs déterminés lors de l'étalonnage s'écartent au-delà d'une mesure donnée à l'avance des facteurs (uRRF) de réponse relatifs universels, les facteurs (uRRF) de réponse relatifs universels étant invariables.

8. Chromatographe (1) en phase gazeuse suivant la revendication 7, **caractérisé en ce que** le dispositif (17) d'évaluation a au moins une interface (30, 33) de données pour la réception des facteurs (uRRF) de réponse relatifs universels.

9. Chromatographe (1) en phase gazeuse suivant la revendication 8, **caractérisé en ce que** l'interface (33) de données est constituée pour recevoir les facteurs (uRRF) de réponse relatifs universels d'un ordinateur (32) à distance pouvant être atteint sur l'Internet (31) .

10. Chromatographe (1) en phase gazeuse suivant la revendication 8 ou 9, **caractérisé en ce que** l'interface (30) est constituée pour lire les facteurs (uRRF) de réponse relatifs universels sans fil ou par fil dans un support (28, 29) de données.

11. Chromatographe (1) en phase gazeuse suivant la revendication 10, **caractérisé en ce que** le support de données est constitué d'un support (29) de données, notamment d'une puce de mémoire, monté sur le dispositif (11) de détection.

12. Chromatographe (1) en phase gazeuse suivant l'une des revendications précédentes, **caractérisé en ce que** le dispositif (17) d'évaluation est constitué pour reconnaître un modèle d'écart, dans le cas où des écarts vont au-delà de la mesure donnée à l'avance pour plusieurs constituants, et pour annoncer une cause d'erreur à l'aide de celui-ci.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03083467 A2
- US 2014260509 A1

- US 2003066803 A1

**In der Beschreibung aufgeführte Nicht-Patentliteratur**

- Guidelines for the quantitative gas chromatography of volatile flavouring substances, from the Working Group on Methods of Analysis of the International Organization of the Flavor Industry (IOFI). *Flavour and Fragrance Journal,* 2011, vol. 26, 297-299

- **K. ROME et al.** Intelligent use of Relative Response Factors in Gas Chromatography-Flame Ionisation Detection. *Chromatography Today,* Mai 2012, 52-56